## Europäisches Patentamt

## European Patent Office

## Office européen des brevets

(11) Veröffentlichungsnummer: **0 129 745**
**A2**

(12) # EUROPÄISCHE PATENTANMELDUNG

(21) Anmeldenummer: **84106368.8**

(22) Anmeldetag: **04.06.84**

(51) Int. Cl.⁴: **A 61 M 5/32**

(30) Priorität: **06.06.83 DE 3320364**

(43) Veröffentlichungstag der Anmeldung:
**02.01.85 Patentblatt 85/1**

(84) Benannte Vertragsstaaten:
**AT BE CH DE FR GB IT LI LU NL SE**

(71) Anmelder: **Henke-Sass, Wolf GmbH**
**Kronenstrasse 16**
**D-7200 Tuttlingen(DE)**

(72) Erfinder: **Schramm, Heinrich Wilhelm**
**Schweriner Strasse 24**
**D-4803 Steinhagen(DE)**

(72) Erfinder: **Wehmeier, Reinhard**
**Schweriner Strasse 19**
**D-4803 Steinhagen(DE)**

(74) Vertreter: **Patentanwälte TER MEER - MÜLLER -**
**STEINMEISTER**
**Artur-Ladebeck-Strasse 51**
**D-4800 Bielefeld 1(DE)**

(54) Injektionsspritze.

(57) Eine Injektionsspritze ist mit einer Biegevorrichtung zum Biegen der Nadel (34) versehen. Die Biegevorrichtung ist beispielsweise durch ein zwischen einem Gehäuse (10) zur Aufnahme einer Zylinderampulle und einer Nadelhalterrung (32) angeordnetes Gelenk (42) gebildet. Wenn das vordere Ende der Nadel (34) in Bezug auf das Gehäuse (10) abgewinkelt wird, um schwer zugängliche Injektionsstellen besser zu erreichen, wird die Nadel durch die Biegevorrichtung gleichmäßig durchgebogen und an beiden Enden des gebogenen Abschnitts geführt. Hierdurch wird die Gefahr verringert, daß die Nadel beim Biegen oder bei der Injektion abbricht.

FIG. 3

EP 0 129 745 A2

INJEKTIONSSPRITZE

Die Erfindung betrifft eine Injektionsspritze mit einem im wesentlichen zylindrischen Gehäuse zur Aufnahme einer Zylinderampulle, einer Nadelhalterung am vorderen Ende des Gehäuses, einer die Nadelhalterung und das vordere Ende des Gehäuses in einer Führung durchdringenden Nadel und einer auf der Nadel befestigten Hülse zur Anbringung an der Nadelhalterung.

Derartige Injektionsspritzen werden beispielsweise in der Zahnmedizin zur Injektion von Anästhetika und dergleichen eingesetzt.

Eine herkömmliche Injektionsspritze der genannten Art ist beispielsweise aus der DE-PS 30 30 239 bekannt. Bei herkömmlichen Injektionsspritzen ist die Nadel durch die an der Nadelhalterung zur befestigende Hülse koaxial in bezug auf das Gehäuse festgelegt. Dies hat den Nachteil, daß bei einer Injektion in die Mundhöhle bestimmte Stellen, wie beispielsweise innere Bereiche des Zahnfleisches, nicht oder nur schwer zu erreichen sind. In solchen Fällen wird üblicherweise das vordere Ende der Nadel in geeigneter Weise gebogen. Das Biegen der Injektionsnadel ist jedoch sehr umständlich, da die Nadel dabei steril bleiben muß. In jüngerer Zeit finden zunehmend verhältnismäßig kurze Injektionsnadeln Verwendung. Zum Biegen dieser kurzen Nadeln ist eine hohe Kraft erforderlich, und es besteht die Gefahr, daß die Nadel beim Biegen abgebrochen wird, da sich die Biegebeanspruchung am vorderen Ende der Hülse konzentriert. Ein weiterer Nachteil besteht darin, daß auch während der Injektion Biegebeanspruchungen in dem gekrümmten Abschnitt der Nadel auftreten, so daß die Nadel sich weiter verbiegt und die Injektion nicht korrekt ausgeführt werden kann.

Der Erfindung liegt die Aufgabe zugrunde, eine Injektionsspritze zu schaffen, die es gestattet, die Nadel in
einfacher Weise zu biegen, ohne daß die Gefahr besteht, daß
die Nadel abbricht.

Diese Aufgabe wird erfindungsgemäß gelöst durch eine am
vorderen Ende der Injektionsspritze befestigte oder ankuppelbare Nadel-Biegevorrichtung, die einen Abschnitt der
Nadel außerhalb des Gehäuses in einer in bezug auf die
Achse des Gehäuses abwinkelbaren Führung aufnimmt.

Erfindungsgemäß wird das Biegen der Nadel durch die in
bezug auf das Gehäuse abwinkelbare Führung der Biegevorrichtung bewirkt, ohne daß die Nadel hierzu unmittelbar
berührt werden müßte. Der Abschnitt der Nadel, der beim
Biegen gekrümmt wird, ist durch die Führung in der Biegevorrichtung fest vorgegeben. Die Krümmung der Nadel verteilt sich daher gleichmäßig auf diesen vorgegebenen Abschnitt, so daß übermäßige Biegebeanspruchungen, die
zum Bruch der Nadel führen könnten, vermieden werden.

Bevorzugt bleibt die Biegevorrichtung auch während der Injektion mit der Injektionsspritze verbunden. Dies hat den
Vorteil, daß die Biegevorrichtung dem abgewinkelten geraden
Endabschnitt im Bereich der Nadelspritze auch während der
Injektion Führung gibt, so daß Biegebeanspruchungen im freien
Ende der Nadel und damit ein Ausweichen oder Brechen der
Nadel während der Injektion vermieden werden.

In einer bevorzugten Ausführungsform der Erfindung umfaßt
die Biegevorrichtung ein Gelenk zwischen dem Gehäuse und
der Nadelhalterung der Injektionsspritze. In diesem Fall
können auch die Nadelhalterung und die auf dieser befestigte  Hülse als Teile der Biegevorrichtung angesehen
werden, die dem abgewinkelten Ende der Nadel Führung geben.

0129745

Das Biegen der Nadel erfolgt in diesem Fall in der Weise, daß die auf der Nadel befestigte Hülse in üblicher Weise zusammen mit einer die Nadelspitze steril aufnehmenden Kappe auf die Nadelhalterung aufgeschraubt und die Nadelhalterung sodann mit Hilfe der Kappe in bezug auf das Gehäuse der Injektionsspritze geschwenkt wird.

Bevorzugt ist das Gelenk als Kugelgelenk ausgebildet, dessen Kugel über einen Schaft mit der Nadelhalterung verbunden ist. Die Kugel ist in einem am vorderen Ende des Gehäuses gebildeten Sitz gelagert und wird durch eine Haube in ihrer Position gehalten. Ein von dem Schaft der Nadelhalterung durchlaufener Schlitz gestattet es, die Nadelhalterung zusammen mit der Kugel um beispielsweise 90° zur Seite zu schwenken. Mit Hilfe einer die Haube übergreifenden Spannhülse wird die Kugel durch die Haube fest gegen ihren Sitz gespannt und dadurch in der jeweils gewünschten Position festgelegt. Die Nadel tritt durch eine sich konisch verjüngende Öffnung in das rückwärtige Ende der Kugel ein und erstreckt sich koaxial durch den Schaft der Nadelhalterung.

In einer abgewandelten Ausführungsform der Erfindung kann das Gelenk vor der Nadelhalterung angeordnet sein. Das Gelenk kann aus Stahl hergestellt und damit sterilisierbar und wieder verwendbar sein. Wahlweise kann jedoch das Gelenk auch wie die Nadel nur für eine einmalige Verwendung vorgesehen sein und in kostengünstiger Weise aus Kunststoff hergestellt werden. In letzterem Fall kann das Gelenk auch mit der zur Befestigung der Nadel an der Nadelhalterung dienenden Hülse integriert sein.

In einer weiteren bevorzugten Ausführungsform der Erfindung wird die Biegevorrichtung durch ein in geeigneter Weise gebogenes Rohr gebildet, das auf das freie Ende der Nadel aufschiebbar und über ein Kupplungsstück mit der Hülse ver-

- 4 -                                    0129745

bindbar ist. In diesem Fall biegt sich die Nadel, während sie in das gebogene Rohr eingeführt wird. Das Rohr kann bereits herstellerseitig gebogen werden, wird jedoch vorzugsweise erst vom Benutzer in der gewünschten Weise gebogen.

Die letztgenannte Ausführungsform der Erfindung ist besonders bei Injektionen vorteilhaft, bei denen nur eine geringe Einstichtiefe erforderlich ist. Die Länge des Rohres wird derart gewählt, daß die Nadel nur wenig über das freie Ende des Rohres hinausgeht. Auf diese Weise wird eine stabile Führung der Nadel erreicht, ohne daß die Sicht im Bereich der Einstichstelle beeinträchtigt wird. Aus diesem Grund kann es auch vorteilhaft sein, das Rohr im ungebogenen Zustand lediglich zur Stabilisierung der Nadel zu verwenden, wenn ein Abwinkeln der Nadelspitze nicht erforderlich ist.

Im folgenden werden bevorzugte Ausführungsbeispiele der Erfindung anhand der Zeichnungen, die auch zwei Figuren zum Stand der Technik umfassen, näher erläutert.

Fig. 1    ist eine Gesamtansicht einer herkömmlichen Injektionsspritze mit einer getrennt dargestellten Nadel;

Fig. 2    zeigt in einem vergrößerten Längsschnitt die Nadelhalterung und die Nadel der herkömmlichen Injektionsspritze;

Fig. 3    ist eine Teilansicht einer erfindungsgemäßen Injektionsspritze;

Fig. 4    ist ein vergrößerter Schnitt durch die Nadelhalterung und ein Gelenk der

- 5 -

0129745

erfindungsgemäßen Injektionsspritze;

Fig. 5    ist ein Teilschnitt durch ein abgewandeltes Ausführungsbeispiel
der erfindungsgemäßen Injektionsspritze.

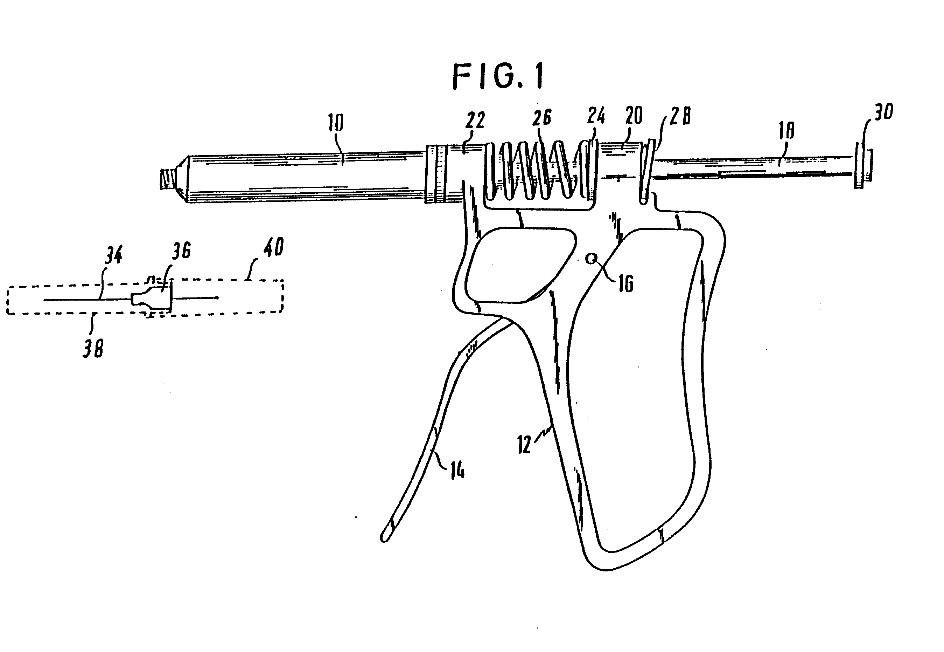

Fig. 1 zeigt den Gesamtaufbau einer als Injektionsspritzpistole ausgebildeten herkömmlichen Injektionsspritze mit
einem Gehäuse 10 zur Aufnahme einer nicht gezeigten Zylinderampulle, einem Griff 12 und einer nachfolgend näher
erläuterten Betätigungseinrichtung.

Die Betätigungseinrichtung umfaßt einen Hebel 14, der um
einen Drehzapfen 16 in Richtung auf den Griff 12 schwenkbar ist und dabei mit seinem oberen Ende eine Kolbenstange 18 nach links in Fig. 1 verschiebt. Die Kolbenstange 18 wird durch zwei mit dem Griff 12 verbundene Rohrabschnitte 20, 22 geführt und beaufschlagt einen Kolben der in dem Gehäuse 10 untergebrachten Zylinderampulle. Der Hebel 14 greift bei Betätigung an einer auf der Kolbenstange 18 angebrachten Scheibe 24 an, die daraufhin verkantet und die Kolbenstange 18 mitnimmt. Nach dem Loslassen des Hebels 14 wird die Scheibe 24 durch eine Feder 26 relativ zu der Kolbenstange 18 gegen den Rohrabschnitt 20 verschoben, und der Hebel 14 kehrt in seine Ausgangsstellung zurück. Die Kolbenstange 18 wird währenddessen durch eine weitere verkantende Scheibe 28 in ihrer vorgerückten Position gehalten. Auf diese Weise kann die Kolbenstange durch mehrmaliges Pumpen mit Hilfe des Hebels 14 nach und nach vorgerückt werden. Zum Zurückziehen der Kolbenstange 18 nach beendeter Injektion wird der obere Rand der Scheibe 28 nach links in Fig. 1 gedrückt, so daß sie die Kolbenstange freigibt, und die Kolbenstange wird an einem Knauf 30 an ihrem rückwärtigen Ende erfaßt und zurückgezogen.

Das Gehäuse 10 weist am vorderen Ende eine mit Außengewinde versehene Nadelhalterung 32 auf, die bei der herkömmlichen Injektionsspritze starr mit dem Gehäuse 10 verbunden ist. Auf einer Nadel 34 ist eine mit Innengewinde versehene Hülse 36 befestigt. Die Nadel ist zunächst in einer in Fig. 1 gestrichelt dargestellten sterilen Verpackung untergebracht, die eine auf die Hülse 36 aufgeschobene Kappe 38 für das vordere Ende der Nadel und eine auf das rückwärtige Ende der Kappe 38 aufgeschobene weitere Kappe 40 für das rückwärtige Ende der Nadel umfaßt.

Vor der Injektion wird zunächst eine Zylinderampulle vom rückwärtigen Ende her in das Gehäuse 10 eingesetzt, das hierzu vorübergehend von dem Rohrabschnitt 22 gelöst wird.

Zur Befestigung der Nadel wird zunächst die Kappe 40 entfernt und die Hülse 36 mit Hilfe der vorderen Kappe 38 auf die Nadelhalterung 32 aufgeschraubt, so daß das rückwärtige Ende der Nadel in die Zylinderampulle eindringt. Sodann wird die vordere Kappe 38 axial nach vorn abgezogen.

Die Länge des rückwärtigen Abschnitts der Nadel ist derart bemessen, daß die Nadel die Stirnwand der Zylinderampulle zwar durchdringt, aber nur wenig in das Innere der Ampulle vorspringt, damit der Hubweg des Kolbens nicht unnötig eingeschränkt wird. Bei herkömmlichen Nadeln beträgt die Länge des nach rückwärts aus der Hülse 36 vorspringenden Abschnitts etwa 10 bis 13 mm.

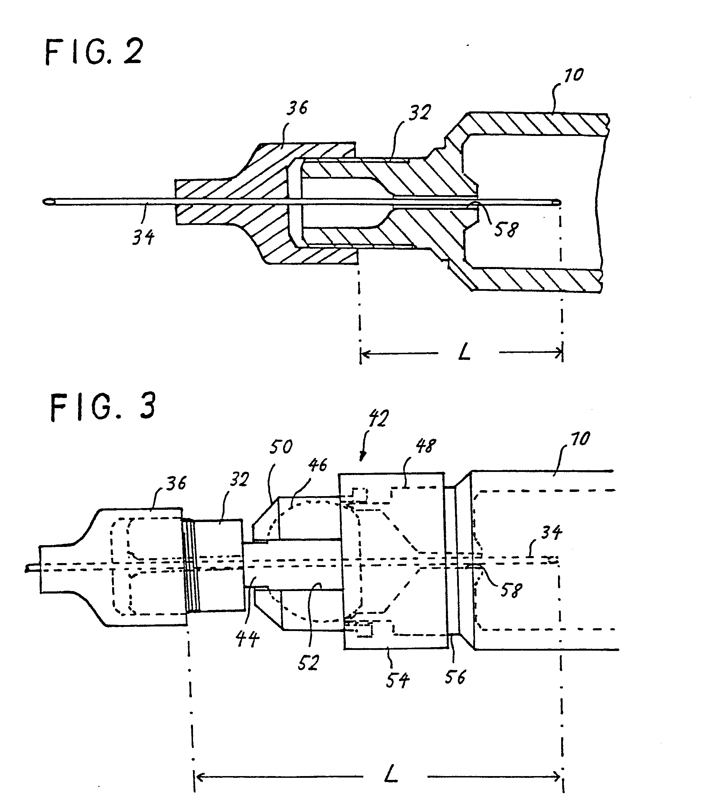

Fig. 2 zeigt einen Schnitt durch das vordere Ende des Gehäuses 10 und durch die Nadelhalterung 32 mit aufgeschraubter Nadel.

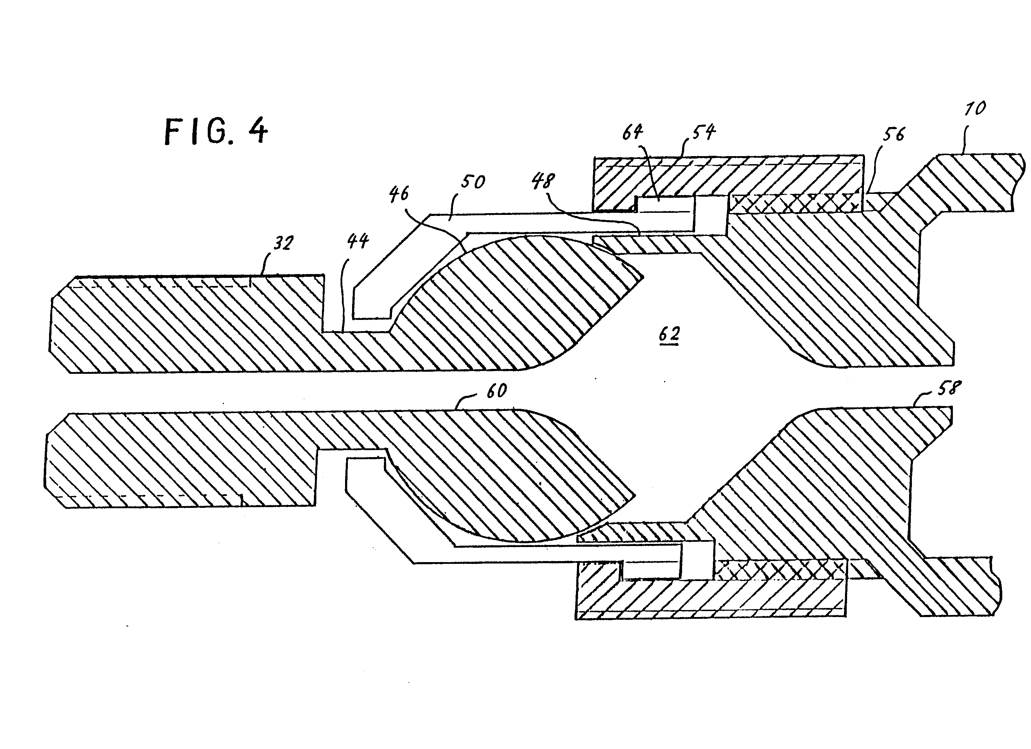

Nunmehr soll mit Bezug auf Fig. 3 und 4 ein bevorzugtes Ausführungsbeispiel der Erfindung erläutert werden.

Die wesentlichen Merkmale der Erfindung betreffen die Ausbildung des vorderen Endes des Gehäuses 10, der Nadelhalterung 32 sowie der Nadel 34 und der Hülse 36. Im übrigen entspricht der Aufbau der erfindungsgemäßen Injektionsspritze beispielsweise der Darstellung in Fig. 1.

Gemäß Fig. 3 ist bei der erfindungsgemäßen Injektionsspritze die Nadelhalterung 32 über ein Gelenk 42 schwenkbar mit dem Gehäuse 10 verbunden. Im gezeigten Beispiel ist das Gelenk 42 ein Kugelgelenk mit einer über einen Schaft 44 starr mit der Nadelhalterung 32 verbundenen Kugel 46, einem am vorderen Ende des Gehäuses 10 ausgebildeten Kugelsitz 48 und einer die Kugel übergreifenden Haube 50. Ein Schlitz 52 erstreckt sich vom Scheitel der Haube 50 aus axial über die Umfangswand der Haube und gestattet eine Schwenkbewegung des Schaftes 44 aus der in Fig. 3 gezeigten axialen Stellung in eine in Richtung auf den Betrachter beispielsweise bis zu 90° abgewinkelten Stellung.Bei geeigneter Dimensionierung der Bauteile kann der Schwenkbereich auch wesentlich mehr als 90° betragen.

Die Haube 50 ist um ihre Längsachse relativ zu dem Gehäuse 10 drehbar, so daß die Nadelhalterung 32 in beliebige Richtungen,bezogen auf den Griff 12 der Injektionsspritze,abgewinkelt werden kann.

Eine auf den Kugelsitz 48 aufgeschraubte Spannhülse 54 erfaßt einen Bund der Haube 50 und ermöglicht es, die Kugel 46 mit Hilfe der Haube 50 fest gegen den Kugelsitz 48 zu spannen und auf diese Weise die Halterung 32 in der gewünschten Position relativ zu dem Gehäuse und dem Griff der Injektionsspritze festzulegen.

Der Kugelsitz 48 weist einen Außengewindeabschnitt 56 auf, auf den die Spannhülse 54 aufschraubbar ist. Wie ein Vergleich mit Fig. 2 zeigt, ist der Durchmesser des Außengewindeabschnitts 56 größer als der der Nadelhalterung 32 der herkömmlichen Injektionsspritze. Auch der nach rückwärts aus der Hülse 36 vorspringende Abschnitt der Nadel 34 ist erfindungsgemäß gegenüber dem entsprechenden Abschnitt der herkömmlichen Nadel verlängert. Die Länge L dieses Abschnitts beträgt erfindungsgemäß etwa 20 mm. Der auf diese Weise vergrößerte axiale Abstand zwischen dem Gehäuse 10 und der Nadelhalterung 32 gestattet es, das Gelenk 42 hinreichend stabil auszuführen.

Der verlängerte rückwärtige Abschnitt der Nadel erstreckt sich wie bei der in Fig. 2 gezeigten herkömmlichen Injektionsspritze durch eine Führungs-Bohrung 58 am vorderen
Ende des Gehäuses 10. Erfindungsgemäß wird die Nadel 34
jedoch zusätzlich durch eine Axialbohrung 60 in der Nadelhalterung 32, dem Schaft 44 und einem Teil der Kugel 46
geführt. Wie deutlich in Fig. 4 zu erkennen ist, ist die
Kugel 46 an ihrem rückwärtigen Ende und der Kugelsitz 48
an seinem vorderen Ende konisch erweitert,und die Übergänge zwischen der konischen Erweiterung und der Bohrung 58
bzw. 60 sind jeweils abgerundet. Hierdurch werden scharfe
Knickstellen in der Nadel vermieden, wenn die Nadel beim
Schwenken der Nadelhalterung 32 gebogen wird. Die Krümmung
der Nadel verteilt sich gleichmäßig über den durch die
konischen Erweiterungen der Kugel 46 und des Kugelsitzes 48
gebildeten Hohlraum  62.

Die Spannhülse 54 ist nur in ihrem rechten Abschnitt in Fig. 4
mit Innengewinde zum Aufschrauben auf den Kugelsitz versehen
und ist in ihrem linken Abschnitt freigestochen, so daß sie
den Bund 64 der Haube 50 frei drehbar aufnimmt. Auch wenn die
Spannhülse 54 von dem Kugelsitz 48 abgeschraubt wird, ist
daher die Haube 50 und damit auch die Kugel 46 und die Nadelhalterung 32 an der Spannhülse festgelegt. Der Bund 64 ist
jedoch mit einem Außengewinde versehen und kann durch das
Innengewinde der Spannhülse 54 geschraubt werden, wenn die
Haube 50 von der Spannhülse getrennt werden soll. Nach dem
Lösen der Haube 50 kann auch die Kugel 46 aus der Haube entnommen werden, indem der Schaft 44 durch den Schlitz 52 geführt wird.

Bei dem gezeigten Ausführungsbeispiel ist das Gelenk 42 derart gestaltet, daß sein Außendurchmesser stufenweise von dem
Durchmesser des Gehäuses 10 auf den Durchmesser der Nadelhalterung 32 abnimmt. Im Zusammenhang mit der Vergrößerung
des Abstands zwischen dem Gehäuse 10 und der Nadelhalterung 32 hat dies den Vorteil, daß die Nadelspitze und damit die Einstichstelle vom Benutzer besser eingesehen werden kann und nicht durch das Gehäuse 10 verdeckt wird.

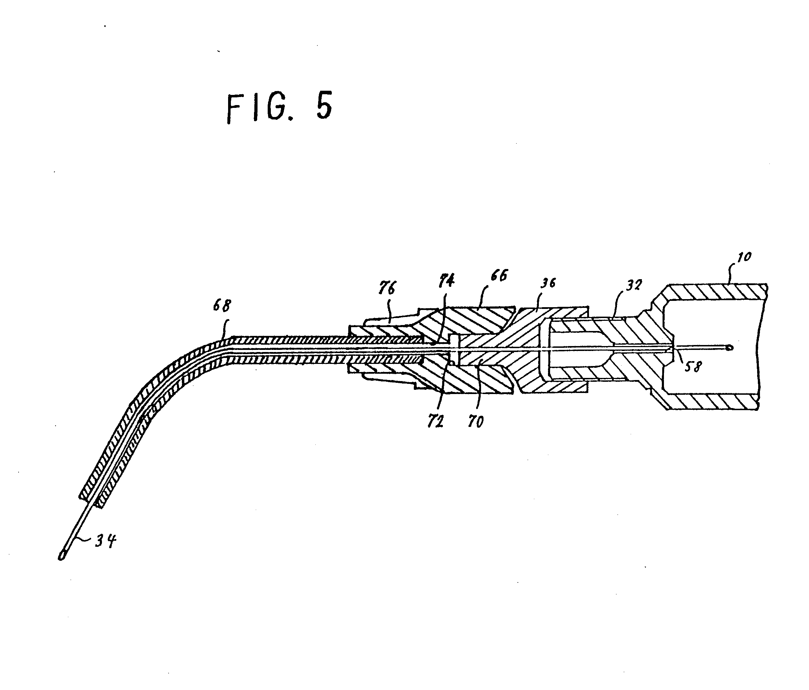

In Figur 5 ist ein abgewandeltes Ausführungsbeispiel der Erfindung veranschaulicht. Bei diesem Ausführungsbeispiel ist die Nadelhalterung 32 in der üblichen Weise starr mit dem Gehäuse 10 verbunden. Die auf die Nadelhalterung 32 aufgeschraubte Hülse 36 trägt ein Kupplungsstück 66, das seinerseits koaxial mit einem Rohr 68 verbunden ist.

Das Kupplungsstück 66 ist beispielsweise aus Kunststoff hergestellt, während das Rohr 68 aus Metall besteht und mit seinem rückwärtigen Ende fest in dem Kupplungsstück 66 eingegossen , eingeklebt oder in sonstiger Weise befestigt ist.

Das Kupplungstück 66 ist auf einen vorderen Abschnitt 70 der Hülse 36 aufgesteckt und weist eine den Abschnitt 70 aufnehmende Sackbohrung auf, an deren Boden sich eine axiale Bohrung 74 befindet, durch die die Nadel 34 in das Rohr 68 eintritt. An seinem verjüngten vorderen Ende ist das Kupplungsstück 66 auf seinem Umfang mit axial verlaufenden Versteifungsrippen 76 versehen.

Die aus Kupplungsstück und Rohr gebildete Einheit wird beispielsweise wie folgt als Biegevorrichtung verwendet. Nachdem die Einheit zunächst steril aus der Verpackung entnommen wurde, wird das Rohr 68 mit Hilfe eines Werkzeugs in gewünschter Weise gebogen, ohne daß dabei das Innere des Rohres infiziert würde. Sodann wird die bereits auf der Injektionsspritze montierte Nadel 34 durch die axiale Bohrung 74 in das Rohr 68 eingeführt, bis der Abschnitt 70 der Hülse 36 mit der Sackbohrung 72 in Eingriff steht. Die verhältnismäßig biegsame Nadel folgt dabei der Krümmung des formstabileren Rohres 68 und wird auf diese Weise gebogen.

Wahlweise ist es jedoch auch möglich, die Nadel vor dem Biegen des Rohres 68 in das Rohr einzuführen und anschließend gemeinsam mit der Nadel zu biegen. Auch in diesem Fall verhindert das Rohr 68, daß die Nadel infiziert oder abgebrochen wird. Die Einheit aus Kupplungsstück und Rohr wird ebenso wie die Nadel nach einmaligem Gebrauch weggeworfen. Wahlweise kann diese Einheit jedoch auch aus sterilisierbarem Material hergestellt und mehrfach verwendbar sein.

– 12 –

PATENTANSPRÜCHE

1. Injektionsspritze mit einem im wesentlichen zylindrischen Gehäuse zur Aufnahme einer Zylinderampulle, einer Nadelhalterung am vorderen Ende des Gehäuses, einer die Nadelhalterung und das vordere Ende des Gehäuses in einer Führung durchdringenden Nadel und einer auf der Nadel befestigten Hülse zur Anbringung an der Nadelhalterung, g e k e n n z e i c h n e t durch eine am vorderen Ende der Injektionsspritze befestigte oder ankuppelbare Nadel-Biegevorrichtung (42;66,68), die einen Abschnitt der Nadel (34) außerhalb des Gehäuses (10) in einer in bezug auf die Achse des Gehäuses abwinkelbaren Führung (60;68) aufnimmt.

2. Injektionsspritze nach Anspruch 1, dadurch g e k e n n z e i c h n e t, daß die Biegevorrichtung ein Gelenk (42) zwischen dem Gehäuse (10) und der Nadelhalterung (32) umfaßt.

3. Injektionsspritze nach Anspruch 2, dadurch g e k e n n z e i c h n e t, daß das Gelenk ein Kugelgelenk (42) mit einer über einen Schaft (44) mit der Nadelhalterung (32) verbundenen, von der Nadel (34) durchlaufenen Kugel (46), einem am vorderen Ende des Gehäuses (10) ausgebildeten Kugelsitz (48) und einer die Kugel (46) übergreifenden Haube (50) ist, die die Kugel (46) in ihrer Position auf dem Kugelsitz (48) hält und den Schaft (44) in einem Schlitz (52) schwenkbar aufnimmt.

4. Injektionsspritze nach Anspruch 3, g e k e n n z e i c h n e t durch eine mit Innengewinde versehene, auf einen Außengewindeabschnitt (56) des Kugelsitzes (48) aufschraubbare, einen äußeren Bund (64) der Haube (50) erfassende Spannhülse (54) zum Spannen der Haube (50) und der Kugel (46) gegen den Kugelsitz.

5. Injektionsspritze nach Anspruch 4, dadurch g e k e n n - z e i c h n e t, daß der Bund (64) der Haube (50) mit einem Außengewinde entsprechend dem Innengewinde der Spannhülse (54) versehen ist und daß der Außendurchmesser der Haube (50) im übrigen überall kleiner als das Innenmaß der Spannhülse ist.

6. Injektionsspritze nach einem der Ansprüche 3 bis 5, insbesondere in der Form einer Injektionsspritzpistole, dadurch g e k e n n z e i c h n e t, daß die Haube (50) relativ zu dem Gehäuse (10) um ihre Längsachse drehbar ist.

7. Injektionsspritze nach einem der Ansprüche 2 bis 6, dadurch g e k e n n z e i c h n e t, daß die Länge L des Abschnitts der Nadel (34) vom rückwärtigen Ende der Hülse (36) bis zum rückwärtigen Ende der Nadel mehr als 16 mm, vorzugsweise etwa 20 mm beträgt.

8. Injektionsspritze nach einem der Ansprüche 3 bis 7, dadurch g e k e n n z e i c h n e t, daß sich die Nadel (34) zwischen einer Führung (60) in dem Schaft (44) und der Kugel (46) und der Führung (58) am vorderen Ende des Gehäuses (10) durch einen Hohlraum (62) erstreckt, der durch konische Erweiterungen am rückwärtigen Ende der Kugel (46) und am vorderen Ende des Kugelsitzes (48) gebildet wird.

9. Injektionsspritze nach Anspruch 8, dadurch g e - k e n n z e i c h n e t, daß die Führungen (58,60) durch Bohrungen gebildet sind, die jeweils in abgerundeter Form in die konische Erweiterung des Kugelsitzes (48) bzw. der Kugel (46) übergehen.

10. Injektionsspritze nach Anspruch 1, dadurch g e - k e n n z e i c h n e t, daß die Biegevorrichtung ein gebogenes oder biegbares Rohr (68) umfaßt, das formstabiler als die Nadel (34) ist und das auf das vordere Ende der Nadel aufschiebbar und über ein Kupplungsstück (66) mit der auf der Nadel befestigten Hülse (36) verbindbar ist.

0129745

FIG. 1

# FIG. 2

# FIG. 3

FIG. 4

## FIG. 5